(19) Europäisches Patentamt / European Patent Office / Office européen des brevets

(11) EP 4 242 319 A1

(12) EUROPEAN PATENT APPLICATION
published in accordance with Art. 153(4) EPC

(43) Date of publication:
13.09.2023 Bulletin 2023/37

(21) Application number: 21889627.2

(22) Date of filing: 05.11.2021

(51) International Patent Classification (IPC):
*C12P 7/52* (2006.01)    *C12P 7/42* (2006.01)
*C12N 15/70* (2006.01)    *C12N 9/88* (2006.01)
*C12N 9/02* (2006.01)

(52) Cooperative Patent Classification (CPC):
C12N 9/0004; C12N 9/88; C12N 15/70; C12P 7/42;
C12P 7/52

(86) International application number:
PCT/KR2021/016057

(87) International publication number:
WO 2022/098162 (12.05.2022 Gazette 2022/19)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 05.11.2020  KR 20200147146
05.11.2020  KR 20200147147

(71) Applicant: Lg Chem, Ltd.
Seoul 07336 (KR)

(72) Inventors:
• LEE, Kyung Muk
Daejeon 34122 (KR)
• KIM, Jae Hyung
Daejeon 34122 (KR)
• HUH, In Young
Daejeon 34122 (KR)

(74) Representative: Goddar, Heinz J. et al
Boehmert & Boehmert
Anwaltspartnerschaft mbB
Pettenkoferstrasse 22
80336 München (DE)

Remarks:
The complete document including Reference
Table(s) and the Sequence Listing(s) can be
downloaded from the EPO website

(54) **METHOD FOR PRODUCING 3-HYDROXYPROPIONIC ACID**

(57) Provided is a two-step production method for 3-HP, comprising: a first step of culturing cells at a high concentration; and a second step of producing 3-HP using the high concentration-cultured cells as a catalyst, in which during the two-step culture, the energy and/or coenzyme balance are adjusted to produce 3-HP and/or improve the productivity of 3-HP. The productivity and yield of 3-HP can be improved.

【FIG. 2】

EP 4 242 319 A1

**Description**

[TECHNICAL FIELD]

Cross-reference to related applications

The present application claims the priority based on Korean Patent Application No. 10-2020-0147146 and 10-2020-0147147 filed on November 05, 2020, and the all the contents disclosed in the documents of the corresponding Korean Patent Applications are incorporated by reference as a part of the present description.

The present application relates to a method for producing 3-hydroxypropionic acid (3-HP) and/or a method for improving production ability of 3-HP, and more specifically, relates to a method for producing 3-HP and/or a method for improving productivity of 3-HP, by regulating energy and/or coenzyme balance during culturing that produces 3-HP using cells having 3-HP productivity as a catalyst.

[BACKGROUND ART]

3-hydroxypropionic acid is a platform compound that can be converted into various chemicals such as acrylic acid, methyl acrylate, acrylamide, and the like. Since it was selected as a Top 12 value-added bio-chemical by the US Department of Energy (DOE) in 2004, it has been actively studied in academia and industry.

Production of 3-HP is largely carried out in two methods of a chemical method and a biological method, but in the case of the chemical method, it is pointed out that the initial material is expensive and it is not eco-friendly due to the fact that toxic substances are generated during the production process, and therefore, eco-friendly bio-processes are in the spotlight.

Glucose and glycerol are mainly used as a substrate for 3-HP biosynthesis using a microorganism, but due to low yield and productivity, despite the potential for 3-HP, commercialization has not yet been reached.

[DISCLOSURE]

[TECHNICAL PROBLEM]

Accordingly, the present inventors have developed a fermentation technology capable of increasing the 3-HP production concentration while maintaining high yield and productivity for commercialization of 3-HP.

One example of the present application provides a method for producing 3-hydroxypropionic acid (3-HP) and/or a method for improving productivity of 3-HP, comprising inoculating cells having 3-hydroxypropionic acid (3-HP) production ability into a medium comprising glycerol as a carbon source and producing 3-HP under a condition of activating an electron transport system.

[TECHNICAL SOLUTION]

The present application provides a method of producing 3-hydroxypropionic acid (3-HP) and/or a method of improving productivity of 3-HP, comprising inoculating cells having 3-hydroxypropionic acid (3-HP) production ability into a medium comprising glycerol as a carbon source, and producing 3-HP under a condition of activating an electron transfer system.

More specifically, the method can comprise

(1) inoculating cells having 3-hydroxypropionic acid (3-HP) production ability into a production medium; and
(2) producing 3-HP by culturing the inoculated cells.

The production medium can comprise glycerol as a carbon source. The production medium during inoculating cells may not comprise glucose as a carbon source.

The culturing can be performed under (i) a condition of maintaining dissolved oxygen (DO) in the production medium at a level of 50% or less, (ii) a condition of adding glucose when the dissolved oxygen in the medium is 0.1% or more, or (iii) both (i) and (ii).

In step (2) of producing 3-HP, proliferation of cells may not occur.

In another embodiment, the method can be a 2-step method further comprising a 'growing/proliferating' that isolates the cells from the medium after high concentration culture, before the aforementioned 'producing step' that produces 3-HP by inoculating cells into a production medium and culturing them.

More specifically, the method can further comprise:

(a) producing a high-concentration culture of cells having 3-hydroxypropionic acid (3-HP) production ability in a growth medium; and

(b) isolating the cultured cells from the growth medium,

before the inoculating step of (1).

The cells isolated from the growth medium can be inoculated into the production medium of step (1). The growth medium may not comprise glycerol as a carbon source.

Hereinafter, the present invention will be described in more detail.

The method for production of 3-HP and/or method for improving productivity of 3-HP provided herein comprises inoculating cells having 3-hydroxypropionic acid (3-HP) production ability into a medium comprising glycerol as a carbon source (3-hydroxypropionic acid production medium), and producing 3-HP under a condition that activates an electron transfer system (for example, condition of maintaining dissolved oxygen at a certain level and/or condition of adding glucose).

The 3-hydroxypropionic acid production medium can be used without limitation within a desired range for allowing cells to produce 3-HP without proliferation (cell division, growth or development) of 3-HP producing cells.

In an embodiment, the carbon source of the production medium can be glycerol, but is not limited thereto. In an embodiment, the medium for production can further comprise vitamin B12. In one embodiment, the production medium may not comprise glucose during inoculating cells.

In an embodiment, the medium can be synthetic media or semisynthetic media, but is not limited thereto.

The cells for inoculating can be prepared by isolating cells having 3-hydroxypropionic acid (3-HP) production ability from a high cell concentration culture, but is not limited thereto.

The inoculating concentration (cell concentration at the time of inoculating) of the high concentration cultured cells can be appropriately adjusted or determined by those skilled in the art within a range of a purpose of producing 3-HP. In an embodiment, the inoculating concentration (based on dry cell weight (DCW)/medium volume (L)) can be 1 to 20 g/L, 1 to 16 g/L, 1 to 12 g/L, 1 to 9 g/L, 2 to 20g/L, 2 to 16 g/L, 2 to 12g/L, 2 to 9 g/L, 4 to 20 g/L, 4 to 16 g/L, 4 to 12 g/L, or 4 to 9 g/L, but not limited thereto.

Producing the 3-hydroxypropionic acid can be performed by selecting a culturing method known in the art without limitation in a range of a purpose for producing 3-HP, and in an embodiment, the culturing can be performed by fermentation culturing.

While producing 3-HP, proliferation of the inoculated cells may not occur. In an embodiment, the number of cells at the end of the producing of 3-HP can be 150% or less, 130% or less, 100% or less, 90% or less, or 80% or less, for example, 50 to 150%, 50 to 130%, 50 to 100%, 50 to 90%, 50 to 80%, 70 to 150%, 70 to 130%, 70 to 100%, 70 to 90%, or 70 to 80%, of the number of the inoculated cells, but is not limited thereto.

The condition of activating the electron transfer system (for example, condition of maintaining dissolved oxygen at a certain level and/or condition of adding glucose) can for example, activate production of $NAD^+$ coenzyme and/or activate ATP production, but is not limited thereto.

The condition of activating the electron transfer system, can be:

(i) adding glucose to a medium (for example, medium and/or culture solution for producing 3-HP);

(ii) maintaining (adjusting) dissolved oxygen (DO) in a medium (for example, medium and/or culture solution for producing 3-HP) at a certain level; or

(iii) a combination of (i) and (ii).

In an embodiment, the condition of activating the electron transfer system can be performed by adding glucose to a medium (for example, medium and/or culture solution for producing 3-HP). The glucose addition can be performed when the dissolved oxygen (DO) in the medium is at a certain level or higher, or performed regardless of the dissolved oxygen in the medium (for example, before the dissolved oxygen in the medium after culturing is at a certain level or higher). The glucose can be added for the purpose of energy supply, not for the purpose of cell growth.

When biosynthesis of 3-HP is performed using glycerol, at a certain point in time, the amount of NAD+ and energy are insufficient, the reaction rate decreases, and the biosynthesis of 3-HP may be stopped. Then, DO, which was 0% at the start of culture (fermentation) rises. 3-HP production can be sustained by resupplying cellular $NAD^+$ and ATP by adding glucose as an energy source at the time of DO elevation. The glucose may be added to the medium before and/or after DO in the medium rises to a certain level or higher.

The dissolved oxygen, which is the basis for addition of glucose, can be appropriately selected as needed in a range of purpose of producing 3-HP, and in an embodiment, the time point of adding glucose can be a time point at which the dissolved oxygen in the medium starts to rise from that at the start of culture, and the dissolved oxygen in the medium which is the basis for addition of glucose can be for example, 0.1% or more, 0.5% or more, 1% or more, 3% or more, 5% or more, 10% or more, 15% or more, 20% or more, 25% or more, or 30% or more, but is not limited thereto (the upper limit may be 100%, 75%, or 50%, but is not limited thereto).

In an embodiment, the amount of the glucose to be added (concentration; glucose weight to be added (g)/medium volume (L)) can be 0.01 to 3g/L, 0.05 to 3g/L, 0.1 to 3g/L, 0.3 to 3 g/L, 0.01 to 2g/L, 0.05 to 2 g/L, 0.1 to 2 g/L, 0.3 to 2 g/L, 0.01 to 1 g/L, 0.05 to 1 g/L, 0.1 to 1 g/L, 0.3 to 1 g/L, 0.01 to 0.7 g/L, 0.05 to 0.7 g/L, 0.1 to 0.7 g/L, 0.3 to 0.7 g/L, 0.01 to 0.3 g/L, 0.05 to 0.3 g/L, or 0.1 to 0.3 g/L, but is not limited thereto.

In an embodiment, the method for producing 3-HP under a condition of activating an electron transfer system by adding glucose to a medium for production as above can have an increased production of 3-HP of 30% or more, 40% or more, or 50% or more, for example, 30 to 1000%, 30 to 500%, 30 to 300%, 30 to 100%, 40 to 1000%, 40 to 500%, 40 to 300%, 40 to 100%, 50 to 1000%, 50 to 500%, 50 to 300%, or 50 to 100%, compared to the control group in which glucose is not added, but is not limited thereto.

In other example, the condition of activating the electron transport system can be performed by maintaining (adjusting) dissolved oxygen (DO) at a certain level.

The "certain level" of DO can be determined without limitation within a range of purpose for producing 3-HP, and for example, it can be at a level of 50% or less, 30% or less, 20% or less, 10% or less, 1 to 50%, 1 to 30%, 1 to 20%, 1 to 10%, 3 to 50%, 3 to 30%, 3 to 20%, 3 to 10%, 5 to 50%, 5 to 30%, 5 to 20%, or 5 to 10%, but is not limited thereto.

In one embodiment, the maintaining of dissolved oxygen can be performed by one or more selected from the group consisting of stirring speed adjustment, air supply adjustment, and pressure adjustment, and the like during culturing, but is not limited thereto. For example, when the dissolved oxygen in the medium or culture, (1) becomes lower than the target range, one or more of stirring speed, air supply and pressure are increased, and (2) when it becomes higher than the target range, one or more of stirring speed, air supply and pressure are decreased, and thereby, the dissolved oxygen in the medium or culture can be maintained in a certain range, but is not limited thereto.

In an embodiment, the method for producing 3-HP under a condition of activating an electron transfer system by maintaining (adjusting) the dissolved oxygen can have an increased production of 3-HP of 30% or more, 50% or more, 70% or more, 30 to 1000%, 30 to 500%, 30 to 300%, 30 to 100%, 50 to 1000%, 50 to 500%, 50 to 300%, 50 to 100%, 70 to 1000%, 70 to 500%, 70 to 300%, or 70 to 100%, compared to the control group not comprising the condition of maintaining dissolved oxygen, but is not limited thereto.

In the method for production of 3-HP and/or method for improving productivity of 3-HP provided in the present description, the cells having 3-hydroxypropionic acid production ability inoculated into the production medium of (1) can be cells isolated from the growth medium after high concentration culture in a growth medium.

For this, the method can further comprise isolating 3-hydroxypropionic acid producing cells after high concentration culture of producing cells for inoculating into the production medium, before the step of producing 3-HP.

More specifically, the method can further comprise:

(a) high concentration culturing cells having 3-hydroxypropionic acid (3-HP) production ability in a growth medium; and
(b) isolating the cultured cells from the growth medium,

before the (1) inoculating.

Then, the cells isolated from the growth medium can be used as cells having 3-hydroxypropionic acid production ability inoculated into the production medium of (1). The growth medium may not comprise glycerol as a carbon source.

In the present description, the 3-hydroxypropionic acid producing cells (can be used interchangeably with 'cells having 3-hydroxypropionic acid production ability') can be selected from microorganisms capable of producing 3-HP from the carbon source (for example, glycerol) in the production medium, for example, microorganisms consisting of *Escherichia* sp. strain (*E. coli,* etc.), Pseudomonas sp., Enterobacteria sp., Brevibacterium sp., Corynebacterium sp., Klebsiella sp., Citrobacter sp., Clostridium sp., Streptomyces sp., Bacillus sp., Lactobacillus sp., Pseudomonas sp., Saccharomyces sp. and Aspergillus sp., but are not limited thereto. In one specific example, the 3-hydroxypropionic acid producing cells can be E. coli.

In an embodiment, the 3-hydroxypropionic acid producing cells can comprise a gene encoding one or more (for example, 1 or both) proteins selected from the group consisting of glycerol dehydratase and aldehyde dehydrogenase. In an embodiment, the 3-HP producing cells can further comprise a gene (gdrAB) encoding glycerol dehydratase reactivase (GdrAB). In an embodiment, the 3-HP producing cells can be cells capable of additionally biosynthesizing vitamin B12.

The glycerol dehydratase can be encoded by dhaB (GenBank accession no. U30903.1) gene, but is not limited thereto. The dhaB gene can be an enzyme derived from Klebsiella pneumonia, but is not limited thereto. The gene encoding glycerol dehydratase can comprise a gene encoding dhaB1, dhaB2 and/or dhaB3. The glycerol dehydratase protein and gene encoding thereof can comprise a mutation of the gene and/or amino acid sequence within a range that maintains the enzymatic activity of decomposing glycerol into 3-hydroxypropanal (3-HPA) and water ($H_2O$).

The gene (aldH) encoding aldehyde dehydrogenase (ALDH) can be aldH (GenBank Accession no. U00096.3;

EaldH) gene derived from Escherichia coli or E. coli K12 MG1655 cell line, puuC gene derived from K. pneumonia and/or KGSADH gene derived from Azospirillum brasilense, but is not limited thereto. The aldehyde dehydrogenase protein and gene encoding thereof can comprise a mutation of the gene and/or amino acid sequence within a range that maintains the activity to produce 3-HP from 3-HPA.

The 3-HP producing cells can comprise a gene encoding one or more, two or more or all 3 kinds selected from the group consisting of glycerol dehydratase, aldehyde dehydratase, and glycerol dehydratase reactivase, or a recombinant vector comprising the gene.

The recombinant vector can be used by substituting a promoter, a regulation site within a range of purpose to express a gene encoding one or more, two or more or all 3 kinds selected from the group consisting of glycerol dehydratase, aldehyde dehydratase, and glycerol dehydratase reactivase in cells by a method known in the art.

The high concentration culture can be performed by using a method known in the art without limitation within a range of purpose to secure 3-HP producing cells in quantity, and in an embodiment, the culture can be performed by fed-batch culture.

In an embodiment, the fed-batch culture can be performed by a pH-stat method, a continuous feeding culture method, or a combination thereof. In one embodiment, when the fed-batch culture of the pH stat method is performed, glucose can be added at a concentration of 1 to 5g/L. In one embodiment, when the fed-batch culture of the continuous feeding culture method is performed, glucose can be injected at a rate of 10 to 20g/L/h.

In an embodiment, during the high concentration culture, pH can be maintained as 5 to 7.5, 5 to 7, 5.5 to 7.5, 5.5 to 7, 6 to 7.5 or 6.5 to 6, but is not limited thereto.

In an embodiment, the carbon source included in the growth medium during the high concentration culture can be a monosaccharide, a disaccharide and/or a polysaccharide without limitation within a range of purpose for high concentration culture. For example, the carbon source can be one or more, two or more, three or more, four or more, five or more, ten or more, or a combination of all 11 kinds, selected from the group consisting of glucose, fructose, galactose, mannose, arabinose, xylose, ribose, sucrose, maltose, lactose and cellobiose. The growth medium may not comprise glycerol as a carbon source.

In an embodiment, the cell concentration after high concentration culture can have OD600 (optical density (O.D.) measured at 600nm) of 10 or more, 50 or more, 100 or more, 150 or more, 200 or more, 10 to 500, 10 to 400, 10 to 300, 10 to 250, 50 to 500, 50 to 400, 50 to 300, 50 to 250, 100 to 500, 100 to 400, 100 to 300, 100 to 250, 150 to 500, 150 to 400, 150 to 300, 150 to 250, 200 to 500, 200 to 400, 200 to 300, or 200 to 250, but is not limited thereto.

Separation of the cells from the high concentration culture, can comprise one or more, two or more, or all of the steps selected from the group consisting of centrifuging cells, removing supernatant, and resuspending pellets with buffer. In an embodiment, the buffer can be PBS (Phosphate buffered saline), but is not limited thereto.

The method for production of 3-HP and/or method for improving productivity of 3-HP provided in the present description can have the 3-HP yield of 80% or more, 85% or more, 90% or more, 93% or more, or 95% or more, but is not limited thereto. The 3-HP yield can be calculated by comparing the 3-HP production in the medium (culture) to the amount of glycerol used in the medium (production medium) in producing the 3-HP, and in an embodiment, it can be calculated using Equation 1 below.

[Equation 1]

Yield (%) = [(Final 3-HP (g))/{(Glycerol before culture (g)) - (Glycerol after culture (g))}] * 100

The 3-HP production ability of the method for producing 3-HP and/or method for improving productivity of 3-HP provided by the present invention can be 2.0 g/L/h or more, 2.5 g/L/h or more, 2.0 to 60 g/L/h, 2.0 to 40 g/L/h, 2.0 to 20 g/L/h, 2.0 to 10 g/L/h, 2.5 to 60 g/L/h, 2.5 to 40 g/L/h, 2.5 to 20 g/L/h, or 2.5 to 10 g/L/h, but is not limited thereto.

Glycerol can be converted into 3-HPA, and then reduced into 1,3-propanediol (1,3-PDO) or oxidized into 3-HP. The method for producing 3-HP and/or method for improving productivity of 3-HP provided in the present description may not produce 1,3-PDO. Accordingly, by not producing 1,3-PDO, the yield of 3-HP can be improved.

In addition, the method for producing 3-HP and/or method for improving productivity of 3-HP provided in the present description can significantly reduce the content of byproducts generated during 3-HP production, such as acetate, lactate, and the like, and remaining glycerol.

Provided is a culture of a 3-hydroxypropionic acid producing strain with a high 3-hydroxypropionic acid content and a low content of byproducts.

The culture can have 3-hydroxypropionic acid of 60g/L or more, 62g/L or more, 65g/L or more, 67g/L or more, 70g/L or more, 72g/L or more, 75g/L or more, 77g/L or more, 78g/L, 79g/L or more, 80g/L or more, 81g/L or more, 82g/L or more, 83g/L or more, 84g/L or more, 85g/L or more, 86g/L or more, 87g/L or more, 88g/L or more, 89g/L or more, or 90g/L or more, based on the total culture (the upper limit may be selected among 85 to 1000g/L without limitation, and for example, it may be 1000g/L, 500g/L, 200g/L, 100g/L, or 95g/L, but is not limited thereto).

In addition, the culture can have one or more characteristics selected from among the following, based on the total culture:

an acetate concentration of 0.25%(w/v) or less, 0.2%(w/v) or less, 0.15(w/v) or less, or 0.1%(w/v) or less;
a lactate concentration of 0.15%(w/v) or less, 0.1%(w/v) or less, 0.05%(w/v) or less, 0.03%(w/v) or less, 0.01 %(w/v) or less, or not present;
a PDO (1,3-propanediol) concentration of 0.25%(w/v) or less, 0.2%(w/v) or less, 0.15(w/v) or less, 0.1%(w/v) or less, 0.07%(w/v) or less, or 0.05%(w/v) or less; and
a glycerol concentration of 0.45%(w/v) or less, 0.4%(w/v) or less, 0.35%(w/v) or less, 0.3%(w/v) or less, 0.25%(w/v) or less, 0.2%(w/v) or less, 0.15(w/v) or less, or 0.1%(w/v) or less. Then, the lower limit of each range may include 0 (not present), or be 0.000001%(w/v), 0.00001%(w/v), 0.0001%(w/v), 0.001%(w/v), or 0.005%(w/v), but is not limited thereto.

In one specific example, the culture can be obtained by the aforementioned method for producing 3-HP and/or method for improving productivity of 3-HP, that is,

(1) inoculating cells having 3-hydroxypropionic acid (3-HP) production ability into a production medium; and
(2) culturing the inoculated cells to produce 3-HP,

but is not limited thereto.

Then, the cells having 3-hydroxypropionic acid production ability inoculated into a production medium in (1) above can be separated from the growth medium after high concentration culture in a growth medium. Therefore, in one specific example, the culture can be obtained by culture of the separated 3-hydroxypropionic acid producing strain obtained by

(1') inoculating cells having 3-hydroxypropionic acid production ability separated from the growth medium after high concentration culture in a growth medium; and
(2') culturing the inoculated cells to produce 3-HP.

The culture can be used for producing 3-hydroxypropionic acid.

Accordingly, also provided is a composition for producing 3-hydroxypropionic acid comprising the culture.

Also provided is a method for producing 3-hydroxypropionic acid comprising separating, recovering and/or purifying 3-hydroxypropionic acid from the composition for producing 3-hydroxypropionic acid.

[ADVANTAGEOUS EFFECTS]

The 3-HP producing method and/or productivity improving method provided by the present invention can significantly improve the 3-HP productivity and yield and can be usefully used for commercialization of 3-HP.

[BRIEF DESCRIPTION OF THE DRAWINGS]

FIG. 1 is a schematic diagram of the biosynthesis pathway for biosynthesizing 3-HP from glycerol.
FIG. 2 is a graph of the result of measuring the production of 3-HP in the second-step culture under the conditions of the control group (Comparative example) and Example 1 and Example 2.

[MODE FOR INVENTION]

Hereinafter, the present invention will be described in more detail by examples. However, the following examples are intended to illustrate the content of the present invention only, but the scope of the present invention is not limited by the following examples.

Unless otherwise specified in the present description, all temperatures are based on degrees Celsius, and nucleic acid sequences are described from the 5' end to the 3' end unless otherwise specified.

**Example 1. Preparation of 3-HP producing strain**

A recombinant vector in which a gene encoding glycerol dehydratase and aldehyde dehydrogenase known to produce 3-hydroxypropionic acid (3-HP) using glycerol as a substrate was introduced was prepared. A 3-HP producing strain was produced by introducing the prepared recombinant vector into E. coli W3110 strain.

Specifically, a gene encoding glycerol dehydratase (dhaB), a gene encoding aldehyde dehydrogenase (aldH) and a gene encoding dehydratase reactivase (gdrAB) were cloned into a plasmid pCDF to produce a recombinant vector for producing 3HP (pCDF_J23101_dhaB_gdrAB_J23100_aldH).

The pCDFDuetJ23 vector used for production of the recombinant vector was a vector in which the promoter portion of the pCDFDuet-1 vector (Novagen, USA) was substituted with J23101 and J23100 promoters. The dhaB (U30903.1; about 2.7 kb; including dhaB1, dhaB2 and dhaB3) and gdrAB genes (about 2.2 kb; gdrA, gdrB) inserted into the vector were amplified in the chromosome of Klebsiella pneumonia (ATCC 25955) using the primers of Table 1 below. Since the dhaB123 and gdrA genes were located side by side on the chromosome of Klebsiella pneumonia, they were amplified together, and since gdrB was located in the opposite direction to dhaB123 and gdrA, only gdrB was amplified separately.

The aldH gene was amplified and separated from the genome of E. coli K12 MG1655 strain using the promoter pair consisting of the nucleic acid sequences of SEQ ID NO: 5 and SEQ ID NO: 6 of Table 1 below. In Table 1 below, the nucleic acid sequences of the primers used for amplification of each gene were shown, and among names, '-F' refers to a forward promoter, and '-R' refers to a reverse promoter.

[Table 1]

| SEQ ID NO | Name | Nucleic acid sequence (5'>3') |
|---|---|---|
| 1 | dhaB-gdrA-F | GAATTCATGAAAAGATCAAAACGATTTGCAGTCCT |
| 2 | dhaB-gdrA-R | AAGCTTGATCTCCCACTGACCAAAGCTGG |
| 3 | gdrB-F | AAGCTTAGAGGGGGCCGTCATGTCGCTTTCACCGCCAG |
| 4 | gdrB-R | CTTAAGTCAGTTTCTCTCACTTAACGGC |
| 5 | aldH-F | ggtaccatgaattttcatcatctggc |
| 6 | aldH-R | catatgtcaggcctccaggcttat |

After amplification of each gene, the dhaB123 and gdrA genes, and the gdrB gene were cloned downstream of the J23101 promoter of the pCDFDuetaJ23 vector using restriction enzymes EcoRI and HindIII, and the restriction enzymes HindIII and AflII, respectively. The aldH was cloned under the J23108 promoter using restriction enzymes KpnI and NdeI. The cloning method of each gene was performed by a method known in the art. The plasmid was introduced into E. coli W3110 (KCCM 40219) by electroporation to prepare a 3HP producing strain.

**Preparation example 1. Two-step cell culture for 3-HP production**

**1-1. High concentration culture of cells (step 1 culture)**

For the prepared 3-HP producing strain, high concentration cell culture was performed by a fed-batch culture method with a 5L fermenter (Working volume 2L).

Specifically, glucose 20g/L, and an antibiotic (streptomycin) for selection 25 mg/L, were added to an MR medium ($KH_2PO_4$ 6.67g, $(NH_4)_2HPO_4$ 4g, $MgSO_4 \cdot 7H_2O$ 0.8g, citric acid 0.8g, and trace metal solution 5mL per 1L; herein, Trace metal solution was 5M HCl 5mL, $FeSO_4 \cdot 7H_2O$ 10g, $CaCl_2$ 2g, $ZnSO_4 \cdot 7H_2O$ 2.2g, $MnSO_4 \cdot 4H_2O$ 0.5g, $CuSO_4 \cdot 5H_2O$ 1g, $(NH_4)_6Mo_7O_2 \cdot 4H_2O$ 0.1g, and $Na_2B_4O_2 \cdot 10H_2O$ 0.02g per 1L) to use as the cell culture medium, and the temperature of 35°C was maintained. pH was maintained by 6.95 using ammonia water, and the dissolved oxygen (DO) was maintained at 20%, raising the stirring speed up to 900 rpm in stages, and aeration was maintained at 1 vvm.

For the fed-batch culture, a pH-stat feeding method was used, and glucose was added at 3g/L.

Cell concentration was measured by measuring optical density (OD) using a UV-Spectrometer according to the lapse of incubation time. At 20 hours after initiation of culture, OD600 was shown to be about 120 (dry cell weight 30 g/L).

After completing the high concentration cell culture, the cell culture solution was centrifuged at 6,000rpm at 4°C for 10 minutes to recover cells. The recovered cells were suspended with PBS (phosphate-buffered saline) and used in the following step.

**1-2. 3-HP production using cells high concentration cultured (step 2 culture)**

The medium for producing 3-HP was prepared by adding glycerol 70g/L and vitamin B12 50$\mu$M in M9 medium without glucose. The cell suspension prepared in the Preparation example 1-1 was inoculated into the medium for producing 3-HP to be the cell inoculum 5g/L (based on the dry cell weight), and the 3-HP producing step was performed in a 5L fermenter (Working volume 2L).
The culture condition for 3-HP production was maintained at the temperature of 35°C and stirring speed of 300rpm, and the aeration was maintained at 1vvm and the pH was maintained at 7.0 using $Ca(OH)_2$.

**Example 2. Glucose supply condition for activation of electron transport system (step 2 culture + glucose addition)**

It is known that ATP is consumed to transport 3-HP synthesized in the cell out of the cell. This ATP synthesis is mainly performed in the electron transport system and ATP synthesis is also possible through substrate-level phosphorylation of glucose. Accordingly, the productivity improving effect of 3-HP was confirmed when ATP was continuously supplied by adding a small amount of glucose.
Specifically, the strain for producing 3-HP isolated after step 1 high concentration culture by the method of Preparation example 1-1 (strain prepared in Preparation example 1), was inoculated under the same condition as the step 2 culture of Preparation example 1-2 to perform culture. However, the culture condition was set to add a glucose solution with a concentration of 700g/L to be 0.3g/L when the dissolved oxygen (DO) during the step 2 culture (in the medium) was measured and it exceeded 20% or more.
As a control group (Comparative example), only the steps of Preparation examples 1-1 to 1-2 were performed and the cell culture and 3-HP production were performed.

**Example 3. Air supply controlling condition for activation of electron transport system (step 2 culture + DO control)**

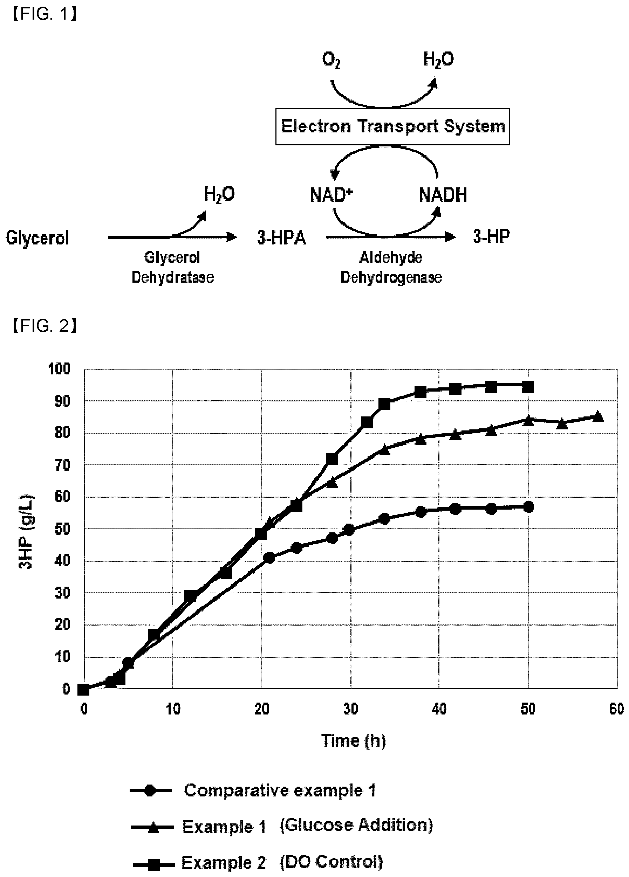

Biosynthesis of 3-HP using glycerol is achieved by glycerol dehydratase converting glycerol to 3-HPA and then aldehyde dehydrogenase converting 3-HPA to 3-HP (FIG. 1). To convert 3-HPA to 3-HP, a coenzyme having oxidizing power, NAD+ is required, and to continuously produce 3-HP, NAD+ converted to NADH should be reproduced. This reproduction process of NAD+ is mostly achieved in the electron transport system. Accordingly, when oxygen is sufficiently supplied to activate the electron transport system, the 3-HP productivity improving effect was confirmed.
Specifically, the strain for producing 3-HP isolated after step 1 culture by the method of Preparation example 1-1, was inoculated under the same condition as the step 2 culture of Preparation example 1-2 and culture was performed. However, in 2 hours after inoculation in the step 2 culture, the culture condition was set to maintain the dissolved oxygen (DO) at 5% by adjusting the stirring speed (rpm). In the present example, the initial stirring speed was started at 300 rpm and after 2 hours, the DO was adjusted by gradually increasing the stirring speed so that the DO was 5%. In other words, when the DO was 5% or less, the stirring speed was increased by 1-2 RPM and when it was 5% or more, the DO was maintained at 5% while lowering it by 1-2 RPM.
As a control group (Comparative example 1), only the steps of Preparation examples 1-1 to 1-2 were performed and the cell culture and 3-HP production were performed. In case of the control group, the initial DO right after inoculation was 0% and at the end of the step 2 culture (40 hours), oxygen consumption was stopped and the DO was increased to 100%.

**Example 4. Comparison of 3-HP production by each culture condition**

While producing 3-HP by the methods of Examples 2 to 3 and the methods of Preparation examples 1-1 to 1-2 (Comparative example 1), the production (g/L) of 3-HP according to the lapse of the step 2 culture time was measured, and the result was shown in FIG. 2.
As can be seen in FIG. 2, when 50 hours elapsed after inoculation for the step 2 culture (3-HP production), in the culture method of Comparative example, only about 56g/L of 3-HP was produced. As Example 2, glucose was added in a small amount to maintain ATP balance, and as a result, 85g/L of 3-HP was produced, and thereby, an increase of 3-HP production of about 1.5 times or more compared to the Comparative example was confirmed (about 52% increase of 3-HP production). When the dissolved oxygen was maintained at a certain level as Example 3 to activate the electron transport system, by the culture method, 95g/L of 3-HP was produced (about 70% increase of 3-HP production). Accordingly, it was experimentally confirmed that the 3-HP productivity could be improved by applying the production method of 3-HP provided by the present invention.

**Example 5. Comparison of 3-HP production according to glucose addition in the step 1 culture and step 2 culture**

3-HP production of the case of step 2 culture where glucose was set to be added when DO exceeded 20% during step 2 culture as Example 2 and the case of step 1 culture where glucose was added (Comparative example 2) was compared.

The Comparative example 2 was prepared as follows: At first, cell growth and 3HP production were simultaneously progressed by adding glucose required for cell growth and a substrate, glycerol to a medium at the same time. The recombinant strain prepared in Example 1 was used, and except for adding glucose and glycerol at the same time, the procedure was the same as Preparation example 1-1 (glucose was added by a fed-batch method and glycerol was added at once during culture). Glucose was additionally added by 1 g/L each time the pH reached 7.0 or higher when all of the initially added glucose was consumed, and 70g/L glycerol was added at 20 and 48 hours of culture.

Culture condition of Comparative example 2: temperature 35°C, pH maintained at 6.95 using ammonia water, stirring speed 500 rpm, Air 1 vvm input

The 3-HP production (g/L) and productivity (g/L/h) of step 1 culture (Example 2) or step 1 culture (Comparative example 2) in Example 2 and Comparative example 2 were measured and shown in Table 2 below:

[Table 2]

| Fermentation method | 3-HP production (g/L) | Productivity (g/L/h) |
|---|---|---|
| Step 1 + glucose addition | 56 | 1.5 |
| Step 2 + glucose addition | 80 | 2.1 |
| (The results in Table 2 are results measured at 38 hours of the step 2 culture (production step) in Example 2 and at 48 hours of the step 1 culture in Comparative example 2, respectively) | | |

As shown in Table 2, in case of Example 2, compared to Comparative example 2, the 3-HP production and productivity were increased. This result shows that the 3-HP production increasing effect according to glucose addition in the step 2 culture was shown much higher.

**Example 6. Comparison of production of impurities in culture**

The content of impurities (acetate, lactate, PDO (1,3-propanediol), and remaining glycerol) in the cultures obtained in Example 2 (step 2 culture + glucose addition) and Example 3 (step 3 culture + DO control) was measured, and it was compared with the case of producing 3-HP by performing only Preparation example 1-1 (step 1 production).

The obtained result was shown in Table 3 below:

[Table 3]

| Impurity | Step 1 production | Example 2 | Example 3 |
|---|---|---|---|
| Acetate | 3 g/L | 1 g/L or less | 1 g/L or less |
| Lactate | 2 g/L | no | no |
| PDO | 3 g/L | 0.5 g/L or less | 0.5 g/L or less |
| Remaining glycerol | 5 g/L or more | 1 g/L or less | 1 g/L or less |
| (The results in Table 3 are results measured at 38 hours of the step 2 culture (production step) and at 48 hours of the step 1 culture in Example 2 and Example 3, respectively) | | | |

As shown in Table 3, in case of Examples 2 and 3, compared to the case of step 1 production, it could be confirmed that the content of impurities such as acetate, lactate, PDO, and remaining glycerol was significant low or did not exist.

**Claims**

1.    A method of producing 3-hydroxypropionic acid (3-HP), comprising

(1) inoculating cells having 3-hydroxypropionic acid (3-HP) production ability into a production medium; and
(2) producing 3-HP by culturing the inoculated cells,
wherein the production medium comprises glycerol as a carbon source, and
the culturing is performed under (i) a condition of maintaining dissolved oxygen (DO) in the production medium at a level of 50% or less, (ii) a condition of adding glucose when the dissolved oxygen in the medium is 0.1% or more, or (iii) both (i) and (ii).

2. The method according to claim 1, wherein in step (2) of producing 3-HP, proliferation of cells does not occur.

3. The method according to claim 1, wherein the producing medium in which the cells are inoculated in step (1) does not comprise glucose as a carbon source.

4. The method according to claim 1, wherein the maintaining the dissolved oxygen of the condition (i) is performed by adjusting the stirring speed.

5. The method according to claim 1, wherein the concentration of the glucose added in the condition (ii) is 0.01 to 3g/L.

6. The method according to any one of claims 1 to 5, wherein the cells having 3-hydroxypropionic acid production ability and inoculated into the production medium in step (1) are isolated from a growth medium after high concentration culture in the growth medium.

7. The method according to claim 6, wherein the cells having production ability of 3-HP comprise a gene encoding at least one protein selected from the group consisting of glycerol dehydratase and aldehyde dehydrogenase.

8. The method according to claim 6, wherein the growth medium does not comprise glycerol as a carbon source.

9. The method according to claim 6, the cell concentration measured by the OD600 value after the high concentration culture is 50 or more.

10. A culture of a strain producing 3-hydroxypropionic acid (3-HP), comprising 3-hydroxypropionic acid at a concentration of 60g/L or more.

11. The culture according to claim 11, having one or more characteristics selected from the following:

    acetate concentration of 0.2%(w/v) or less,
    lactate concentration of 0.1%(w/v) or less,
    PDO (1,3-propanediol) concentration of 0.2%(w/v) or less, and
    glycerol concentration of 0.3%(w/v) or less.

12. A composition for producing 3-hydroxypropionic acid comprising the culture of claim 10 or claim 11.

【FIG. 1】

【FIG. 2】

Time (h)

● Comparative example 1

▲ Example 1 (Glucose Addition)

■ Example 2 (DO Control)

## INTERNATIONAL SEARCH REPORT

| International application No. |
| --- |
| **PCT/KR2021/016057** |

**A. CLASSIFICATION OF SUBJECT MATTER**

**C12P 7/52**(2006.01)i; **C12P 7/42**(2006.01)i; **C12N 15/70**(2006.01)i; **C12N 9/88**(2006.01)i; **C12N 9/02**(2006.01)i

According to International Patent Classification (IPC) or to both national classification and IPC

**B. FIELDS SEARCHED**

Minimum documentation searched (classification system followed by classification symbols)

C12P 7/52(2006.01); C12N 1/21(2006.01); C12P 7/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 3-하이드록시프로피온산 (3-hydroxypropionic acid), 용존산소량 (dissolved oxygen), 글리세롤 (glycerol), 배지 (medium), 포도당 (glucose), 탈수효소 (dehydrogenase)

**C. DOCUMENTS CONSIDERED TO BE RELEVANT**

| Category* | Citation of document, with indication, where appropriate, of the relevant passages | Relevant to claim No. |
| --- | --- | --- |
| X <br> Y <br> A | LI, Y. et al. High production of 3-hydroxypropionic acid in Klebsiella pneumoniae by systematic optimization of glycerol metabolism. Scientific reports. 2016, vol. 6, thesis no. 26932, pp. 1-10. <br>     See abstract; and pages 1 and 5-8. | 10-12 <br> 1-4,6-9 <br> 5 |
| Y | WO 2014-145297 A1 (OPX BIOTECHNOLOGIES, INC.) 18 September 2014 (2014-09-18) <br>     See abstract; and paragraphs [0069] and [0087]. | 1-4,6-9 |
| A | KWAK, S. et al. Biosynthesis of 3-hydroxypropionic acid from glycerol in recombinant Escherichia coli expressing Lactobacillus brevis dhaB and dhaR gene clusters and E. coli K-12 aldH. Bioresource technology. 2013, vol. 135, pp. 432-439. <br>     See entire document. | 1-12 |
| A | WO 2011-094457 A1 (OPX BIOTECHNOLOGIES, INC. et al.) 04 August 2011 (2011-08-04) <br>     See entire document. | 1-12 |

☑ Further documents are listed in the continuation of Box C.    ☑ See patent family annex.

| | |
| --- | --- |
| *   Special categories of cited documents: <br> "A"   document defining the general state of the art which is not considered to be of particular relevance <br> "D"   document cited by the applicant in the international application <br> "E"   earlier application or patent but published on or after the international filing date <br> "L"   document which may throw doubts on priority claim(s) or which is cited to establish the publication date of another citation or other special reason (as specified) <br> "O"   document referring to an oral disclosure, use, exhibition or other means <br> "P"   document published prior to the international filing date but later than the priority date claimed | "T"   later document published after the international filing date or priority date and not in conflict with the application but cited to understand the principle or theory underlying the invention <br> "X"   document of particular relevance; the claimed invention cannot be considered novel or cannot be considered to involve an inventive step when the document is taken alone <br> "Y"   document of particular relevance; the claimed invention cannot be considered to involve an inventive step when the document is combined with one or more other such documents, such combination being obvious to a person skilled in the art <br> "&"   document member of the same patent family |

| Date of the actual completion of the international search | Date of mailing of the international search report |
| --- | --- |
| **11 February 2022** | **14 February 2022** |

| Name and mailing address of the ISA/KR | Authorized officer |
| --- | --- |
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

| INTERNATIONAL SEARCH REPORT | International application No. |
|---|---|
| | **PCT/KR2021/016057** |

**C.    DOCUMENTS CONSIDERED TO BE RELEVANT**

| Category* | Citation of document, with indication, where appropriate, of the relevant passages | Relevant to claim No. |
|---|---|---|
| A | MATSAKAS, L. et al. Biological production of 3-hydroxypropionic acid: an update on the current status. Fermentation. 2018, vol. 4, thesis no. 13, pp. 1-21. <br> See entire document. | 1-12 |

Form PCT/ISA/210 (second sheet) (July 2019)

**INTERNATIONAL SEARCH REPORT**

| International application No. |
|---|
| **PCT/KR2021/016057** |

**Box No. I    Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (July 2019)

**INTERNATIONAL SEARCH REPORT**
Information on patent family members

International application No.

**PCT/KR2021/016057**

| Patent document cited in search report | | | Publication date (day/month/year) | Patent family member(s) | | | Publication date (day/month/year) |
|---|---|---|---|---|---|---|---|
| WO | 2014-145297 | A1 | 18 September 2014 | US | 2015-0044746 | A1 | 12 February 2015 |
| | | | | US | 2016-0130614 | A1 | 12 May 2016 |
| WO | 2011-094457 | A1 | 04 August 2011 | AU | 2010-298004 | A1 | 26 April 2012 |
| | | | | AU | 2010-298004 | B2 | 25 February 2016 |
| | | | | AU | 2011-210852 | A1 | 23 August 2012 |
| | | | | AU | 2011-210852 | B2 | 24 December 2015 |
| | | | | BR | 112012006801 | A2 | 10 April 2018 |
| | | | | CA | 2775390 | A1 | 31 March 2011 |
| | | | | CA | 2775390 | C | 29 June 2021 |
| | | | | CA | 2788045 | A1 | 04 August 2011 |
| | | | | CN | 102695799 | A | 26 September 2012 |
| | | | | CN | 102822347 | A | 12 December 2012 |
| | | | | EP | 2480673 | A1 | 01 August 2012 |
| | | | | EP | 2480673 | B1 | 23 May 2018 |
| | | | | EP | 2529023 | A1 | 05 December 2012 |
| | | | | GB | 2473755 | A | 23 March 2011 |
| | | | | GB | 2473755 | B | 07 September 2011 |
| | | | | GB | 2487866 | A | 08 August 2012 |
| | | | | GB | 2492256 | A | 26 December 2012 |
| | | | | IN | 6617DEN2012 | A | 23 October 2015 |
| | | | | JP | 2013-128494 | A | 04 July 2013 |
| | | | | JP | 2013-505727 | A | 21 February 2013 |
| | | | | JP | 2016-036346 | A | 22 March 2016 |
| | | | | JP | 2016-039825 | A | 24 March 2016 |
| | | | | JP | 6271494 | B2 | 31 January 2018 |
| | | | | KR | 10-2012-0136349 | A | 18 December 2012 |
| | | | | KR | 10-2014-0015136 | A | 06 February 2014 |
| | | | | MX | 2012003604 | A | 12 September 2012 |
| | | | | MX | 2012008700 | A | 05 October 2012 |
| | | | | US | 10100342 | B2 | 16 October 2018 |
| | | | | US | 2013-0071893 | A1 | 21 March 2013 |
| | | | | US | 2013-0122541 | A1 | 16 May 2013 |
| | | | | US | 2014-0045231 | A1 | 13 February 2014 |
| | | | | US | 2015-0056651 | A1 | 26 February 2015 |
| | | | | US | 2015-0344916 | A1 | 03 December 2015 |
| | | | | US | 2017-0114377 | A1 | 27 April 2017 |
| | | | | US | 2019-0119708 | A1 | 25 April 2019 |
| | | | | US | 8883464 | B2 | 11 November 2014 |
| | | | | US | 9388419 | B2 | 12 July 2016 |
| | | | | US | 9428778 | B2 | 30 August 2016 |
| | | | | WO | 2011-038364 | A1 | 31 March 2011 |
| | | | | WO | 2011-094457 | A8 | 04 August 2011 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020200147146
- KR 1020200147147